# EUROPEAN PATENT SPECIFICATION

(11) **EP 2 240 095 B1**
(45) Date of publication and mention of the grant of the patent: **30.09.2015**
(21) Application number: 09707446.2
(22) Date of filing: 06.02.2009
(51) Int. Cl.: A61B 17/28, A61B 17/00, A61B 17/29

(54) **Apparatus for articulating the wrist of a laparoscopic grasping instrument**
Vorrichtung zur Bewegung des Gelenks eines laparoskopischen Greifinstruments
Appareil pour l'articulation du poignet d'un instrument de saisie laparoscopique

(30) Priority: 06.02.2008 US 27231
(43) Date of publication of application: 20.10.2010
(73) Proprietor: Aesculap AG, 78532 Tuttlingen (DE)
(72) Inventor: KERVER, Lawrence, Los Gatos CA 95033 (US); TANG, Brian, Fremont CA 94539 (US); HO, Friedrich, Mountain View CA 94043 (US); NORDELL, Ben, San Mateo CA 94403 (US)
(74) Representative: Winter, Brandl, Fürniss, Hübner, Röss, Kaiser, Polte - Partnerschaft mbB
(86) International application number: PCT/US2009/033443
(87) International publication number: WO 2009/100366

(56) References cited:
- WO-A1-02/080783
- WO-A2-2007/146842
- US-A- 5 456 684
- US-A- 5 456 684
- US-A- 5 549 637
- US-A- 5 766 196
- US-A- 6 077 287
- US-A1- 2006 190 029
- US-A1- 2007 185 518
- US-A1- 2007 250 113

## Description

The invention relates to medical devices for use during laparoscopic procedures. More particularly, the invention relates to an apparatus for articulating the wrist of a laparoscopic grasping instrument.

Laparoscopic surgery, also called minimally invasive surgery (MIS), band aid surgery, keyhole surgery, or pinhole surgery is a modern surgical technique in which operations in the abdomen are performed through small incisions, usually 0.5-1.5 cm, as compared to larger incisions needed in traditional surgical procedures. Laparoscopic surgery includes operations within the abdominal or pelvic cavities, whereas keyhole surgery performed on the thoracic or chest cavity is called thoracoscopic surgery. Laparoscopic and thoracoscopic surgery belong to the broader field of endoscopy.

The key element in laparoscopic surgery is the use of a laparoscope: a telescopic rod lens system, that is usually connected to a video camera (single chip or three chip). Also attached is a fiber optic cable system connected to a cold light source (halogen or xenon), to illuminate the operative field, inserted through a 5 mm or 10 mm cannula to view the operative field. The abdomen is usually insufflated with carbon dioxide gas to create a working and viewing space. The abdomen is essentially blown up like a balloon (insufflated), elevating the abdominal wall above the internal organs like a dome. The gas used is CO2, as it is common to the human body and can be removed by the respiratory system if it absorbs through tissue. It is also non-flammable, which is important due to the fact that electrosurgical devices are commonly used in laparoscopic procedures.

Surgery is performed during a laparoscopic procedure with any of various tools that are typically arranged on one end of a long shaft and that are operable by manipulation of a handle or other actuator positioned at the other end of the shaft.

One area of laparoscopic surgery that is currently the subject of interest is that of electrocauterization. Electrocauterization, also called electric surgery or electrosurgery, is the process of destroying tissue with electricity and is widely used in modern surgery. The procedure is frequently used to stop bleeding of small vessels, larger vessels being ligated, or for cutting through soft tissue, i.e. abdominal fat in a laparotomy or breast tissue in a mastectomy.

US 6,077,87 A discloses a disposable surgical instrument which includes a handle, a rigid stem section extending from the handle and defining a longitudinal axis of the surgical instrument, and a flexible stem section extending from a distal end of the rigid stem section. A surgical tool is connected with a distal end of the flexible stem section. The surgical tool includes a fixed part fixed for movement with the distal end of the flexible stem section, and a movable part supported for pivotal movement relative to the fixed part about a pivot axis. The flexible stem section includes a plurality of relatively pivotable vertebrae or links-arranged between an interface element and the surgical tool.

WO 2007/146842 A discloses an instrument having the feratures of the preamble of claim 1.

US 5,766,196 A shows a medical instrument with a steerable distal end, having a handle for actuating the instrument, a distal operating section for performing a medical procedure, a middle section connecting the handle with the operating section, at least a portion of the middle section being bendable, and steerable means within the bendable portion for permitting the operating section to be controlled in direction at an angle with respect to the axis of the middle section. There are means for rotating the distal operating section into any desired angular position with respect to the longitudinal axis of the middle section. A control member controls the direction of movement of the bendable section in three dimensions whereby the distal end may be positioned in any place on or off of the longitudinal axis of the middle section.

One problem with state of the art of electrocauterization devices for use during a laparoscopic procedure is the limited range of motion provided by the jaws of such device, and the difficulty encountered by a surgeon in positioning such device, and in operating such device through a range of motion, during a laparoscopic procedure.

The invention provides a laparoscopic grasping instrument as defined in claim 1, or any one of the dependent claims.

An embodiment has a set of opposing jaws that can be articulated, both left and right, from centerline. The embodiment also provides a proper bend radius and support for the jaw actuation member and cutter driving member. The bendable support for the drive members in the embodiment comprises tightly wound coil springs.

Another example incorporates a method to control the degree of articulation at the handle of the laparoscopic instrument. The invention incorporates a locking mechanism to prevent motion of the wrist while the user performs other operations on the device. The locking mechanism also includes an indexing feature with which the user can index and choose the necessary amount of angle between preset angles.

### BRIEF DESCRIPTION OF THE DRAWINGS

FIG. 1 is a perspective diagram showing the wrist of a laparoscopic grasping instrument;
FIG. 2 is a plan view showing a wrist of a laparoscopic grasping instrument;
FIG. 3 is a schematic view showing a top cutaway of a wrist articulation control mechanism;
FIG. 4 is a perspective schematic view showing a laparoscopic grasping instrument;
FIG. 5 is another perspective view of a laparoscopic grasping instrument;
FIG. 6 is a perspective schematic view of an indexing mechanism for a laparoscopic grasping instrument;
FIG. 7 is a perspective schematic view of a detent mechanism for a laparoscopic grasping instrument;
FIG. 8 is a perspective schematic view of a detent and indexing mechanism for a laparoscopic grasping instrument;
FIG. 9 is a plan schematic view of a step ball detent mechanism for a laparoscopic grasping instrument;
FIG. 10 is a perspective schematic view of the step ball detent mechanism for a laparoscopic grasping instrument;
FIG. 11 is a second perspective schematic view of the step ball detent mechanism for a .laparoscopic grasping instrument;
FIG. 12 is a perspective schematic view of a push lock mechanism for an articulation control in a laparoscopic grasping instrument;
FIG. 13 is a phantom perspective schematic view of the push lock mechanism for an articulation control mechanism in a laparoscopic grasping instrument;
FIG. 14 is a perspective schematic view of a grab knob for the push lock mechanism in a articulation control for a laparoscopic grasping instrument;
FIG. 15 is a perspective, partially cutaway view of a laparoscopic device, showing a drive member; and
FIG. 16 is a perspective view of a drive assembly for a laparoscopic device blade.

### DETAILED DESCRIPTION OF THE INVENTION

The invention provides a laparoscopic grasping instrument having an apparatus for articulating the wrist of the laparoscopic grasping instrument. The medical instrument has a set of opposing jaws that can be articulated, e.g. 45 degrees or otherwise as desired, both left and right, from centerline. The invention also provides a proper bend radius and support for the jaw actuation member and cutter driving member. The bendable support for the drive members in the embodiment comprises tightly wound coil springs.

An example incorporates a method to control the degree of articulation at the handle of the laparoscopic instrument. The invention incorporates a locking mechanism to prevent motion of the wrist while the user performs other operations on the device. The locking mechanism also includes an indexing feature with which the user can index and choose the necessary amount of angle between preset angles.

The embodiment comprises a medical instrument, preferably for performing a laparoscopic procedure, which comprises a set of pivotal vertebra that are connected to each other by pins or by a snap fit. Each vertebra is adapted to pivot in relation to a device shaft and jaw set, thus allowing left and right articulation. The degree of articulation is controlled by wires or cables that run down both sides of a device wrist. The wires are then routed down the shaft and connected in tension to a control mechanism at a device handle. The cables or wires are used to transfer the forces from the handle to the wrist.

The vertebra form the proper bend radius to allow for a force transfer member, such as a wire, to pass through the wrist without kinking the wire. Furthermore, in one embodiment a tightly wound coil spring is housed within the wrist joints to route said wire. The tightly wound coil spring provides additional support to the wire, such that when the wire is moved from a proximal to a distal direction, it does not buckle or kink.

The control mechanism at the handle consists of a rotating assembly that receives the force transfer members from the wrist. The rotating assembly is pivotally mounted at the handle, and the shape of the control mechanism allows for concentric rotation about the pivot so that the length-wise motion of the wires or cables along the shaft can be controlled, based upon the distance from the pivot to the attachment point of said wires or cables. The angle of articulation is controlled by the distance that the force transfer member moves, which is predetermined by the wrist geometry.

There are several embodiments that comprise a locking and indexing feature.

In a first embodiment, a spring steel is formed into a geometry that deflects when a force is applied, as with a leaf spring. The leaf spring is housed within a circular carrier, with only the deflectable portion of the spring accessible and protruding from a circular carrier. A rotating member with a circular portion removed from its pivot area fits over the circular carrier. A tooth pattern is also removed from along the inner diameter of the circular portion of the rotating member. The rotating member includes arms extending from its center body to which the cable or wires are attached. The leaf-like spring protrudes into the indentations created by the tooth pattern. The angle of articulation is controlled by predetermining the distances between the teeth and the distance from the attachment point of the cable or wires to the pivot point.

In a second embodiment, a spring plunger is mounted within the circular carrier. The spring plunger mates with the indents created by the tooth pattern.

In a third embodiment, the rotating member described above does not have arms extending from its center body. A wing is mounted on top of the rotating member. The wing is then manipulated to control the rotation around the circular carrier.

In a fourth embodiment, a living plastic hinge is mounted near the handle. The living plastic hinge uses a V-shape that fits within a slot of an external housing that surrounds the living hinge. The tip of the V-shape protrudes from each slot. There are a series of slots along the length of the external housing. The housing engages with the cable and wires that control articulation of the wrist. The user can adjust and lock the wrist articulation by first pressing down on the living hinge to disengage the current locked position, then moving the external housing from a proximal to a distal position or vice versa, which then locks by re-engaging with the living hinge at any various predetermined distances set by the slots. These distances determine the angle at which the wrist is articulated.

In an example, the rotating mechanism described above rotates freely around the pivot. When the user has determined the angle of articulation, a button mounted on top of the pivot is depressed, which locks the wrist angle and the rotating mechanism, thus preventing any further movement of both the rotating mechanism and wrist. This can be accomplished using a wedge-like design that is anchored within the pivot pin, which in this embodiment is a tube. A minimum of a single slot is designed into the pivot pin. When the button is depressed, the inherent spring properties of the button flare from the slot. The flaring material uses friction to prevent movement of the rotating mechanism. The button itself remains in place due to a wedge design at the top.

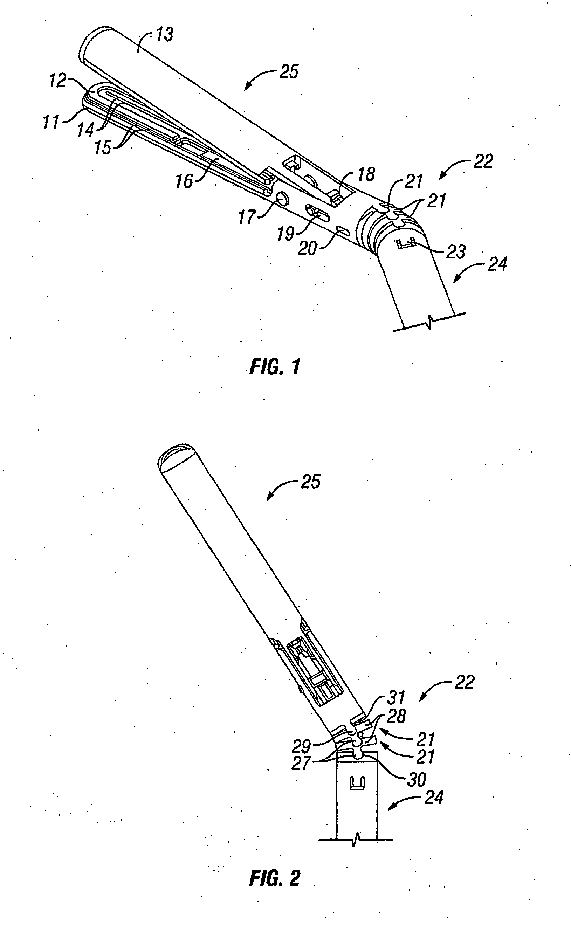

Further to the foregoing discussion, a more detailed explanation of the invention is now provided in connection with FIGS. 1-14.

FIG. 1 is a first perspective view of a laparoscopic device. FIG. 1 is a partial view that shows the main shaft 24 of the device and the jaw assembly 25, which is comprised of an upper jaw 13 and a lower jaw 11. In this embodiment, the upper jaw is pivotable away from and toward the lower jaw about a pivot point 17, which, in this embodiment is comprised of a pin or axle. In other embodiments, the lower jaw may be pivotable as well, but in the embodiment shown in FIG. 1, the lower jaw is fixed. Pivoting of the upper jaw is accomplished by transmitting tension to a jaw activation pin 18, which is moveable, in an activation, slot 19. Typically, tension is applied via a cable attached to the jaw activation pin. Thus, movement of the jaws is accomplished. The jaws themselves are configured for such laparoscopic procedures as electrocautery and tissue severing. Accordingly, as shown in the bottom jaw 11, a distal electrode 12 is provided, embedded in the plastic carrier 15. A second, proximal electrode 15 is also shown. A cutting groove 14 is shown for receiving a blade during a sectioning operation. The blade is not shown in FIG. 1.

During laparoscopic procedures, it is desirable to be able to position the jaws of the device from left to right to achieve the best angle of approach to the tissue to be treated: Key to the invention is the provision of an articulated wrist 22, which is comprised of a plurality of articulation discs or vertebrae 21. The articulation is accomplished by tensioning a pair of cables discussed below and a termination of which is shown in FIG. 1 as a wire, which is soldered or crimped in a groove at a cable termination point 20. Further, FIG. 1 shows a lock for an outside shaft tube or clamping mechanism to hold the wrist to the tube. This is shown by a clamping slot 23.

FIG. 2 is a top or plan view of the laparoscopic device showing the jaws 25 and shaft 24. In particular, the articulated Wrist 22 is shown in greater detail. In this embodiment a plurality of vertebrae comprises interconnected pivotable, hinged disks, where the discs 21 are articulated with one another and comprise a series of ball-like projections 27, which are engaged in complementary grooves 28. The jaw assembly 25 in this embodiment shows a ball-like projection 29, which is engaged in a groove of the articulation disc and the shaft 24 includes a complementary groove 30 for receiving a ball-like projection of an articulating disc. As can be seen in FIG. 2, a cable 31 is shown as well. The cable is a coiled pipe sheath assembly that, in this embodiment, is used to operate a blade within the jaw. The coiled assembly allows the cable to bend with the articulation of the device without kinking, as discussed above.

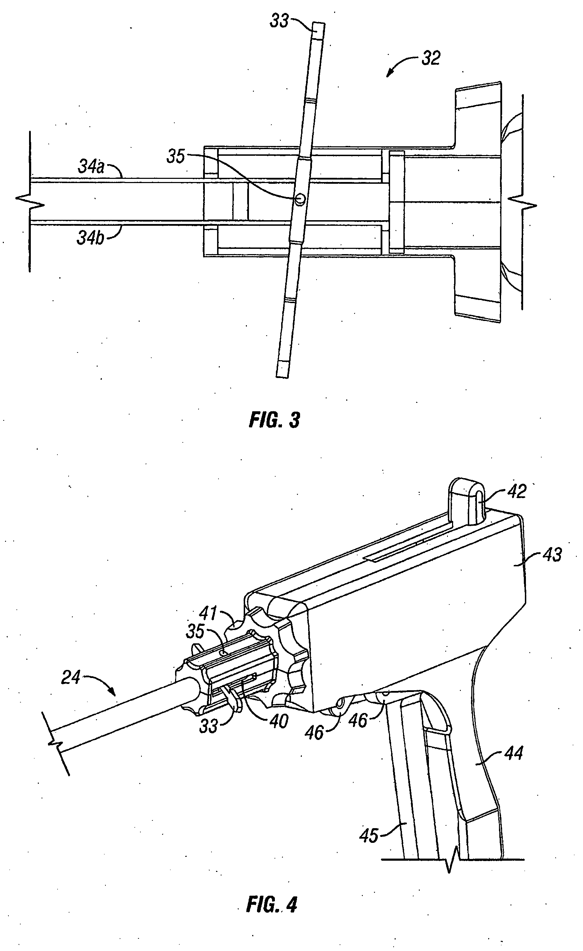

FIG. 3 is a partially cutaway side schematic view of an activation mechanism 32 for operating the articulation joint. In FIG. 3, a wrist articulation control 33 is shown having two finger-actuated blades to pivot the control about a pivot point 35. This pivoting action respectively applies attention to and draws attention from a pair of control cables 34a/34b, which, in this embodiment, are pretensioned Nitinol cables. Those skilled in the art will appreciate that other cable materials may be used. Operation of the wrist articulation control causes one cable to pull on the jaw assembly 25, thus causing the jaw assembly to move left or right as desired. Key to the invention is the provision of the articulation discs which allow such articulation to occur. In the prior art, it is known that such mechanisms as kerfs or other bending mechanisms can be provided. However, these mechanisms are subject both to stress, which reduces their effectiveness over time, and they retain a memory effect, such that there is a tendency for them to return to their initial position, rather the maintain a position desired by the surgeon using the device. The invention herein avoids both of these deleterious effects of operation of the device.

FIG. 4 is a perspective view of a laparoscopic device showing a housing 43 having a handle 44 and a jaw activation trigger 45 that operates a four-bar linkage or other type of linkage 46 to transmit tension through the main shaft 24 and thereby operate the jaws to open and close them as desired. Also shown in FIG. 4 is a blade actuator 42 by which a blade may be drawn through the groove discussed above. A shaft rotator 41 allows the shaft to be rotated about a shaft access, while the wrist articulation control 33 allows the wrist mechanism to be operated. Note in FIG. 4 that the wrist articulation control includes a control slot 40 that both guides and contains the travel of the wrist articulation control 33.

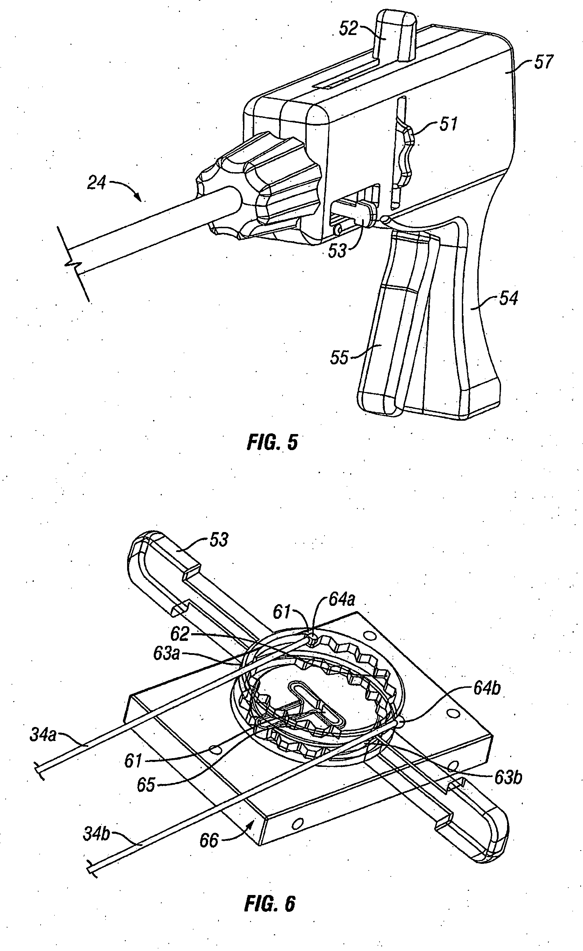

FIG. 5 is a perspective view of a further embodiment in which the shaft rotator 51 is contained within the housing 57. This embodiment also includes a blade actuator 52, a wrist articulation control 53, a handle 54, and a jaw activation trigger 55.

FIG. 6 is a perspective schematic view of the wrist activation control of the laparoscopic device shown in FIG. 5. A base portion 66 supports a ring projection 65 which, in turn, accommodates the control 53. Tensioned cables 34a/34b are shown having termination balls, which provide a cable stop 64a/64b. The cables are threaded through the control actuator 53 through respective grooves 63a/63b. An indexing disc 61 includes a plurality of detents 62. A flat spring 61 is arranged to engage within said detents to provide a stop mechanism to secure the jaws in a selected position by preventing movement of the articulation control 53, except when desired by a user of the device.

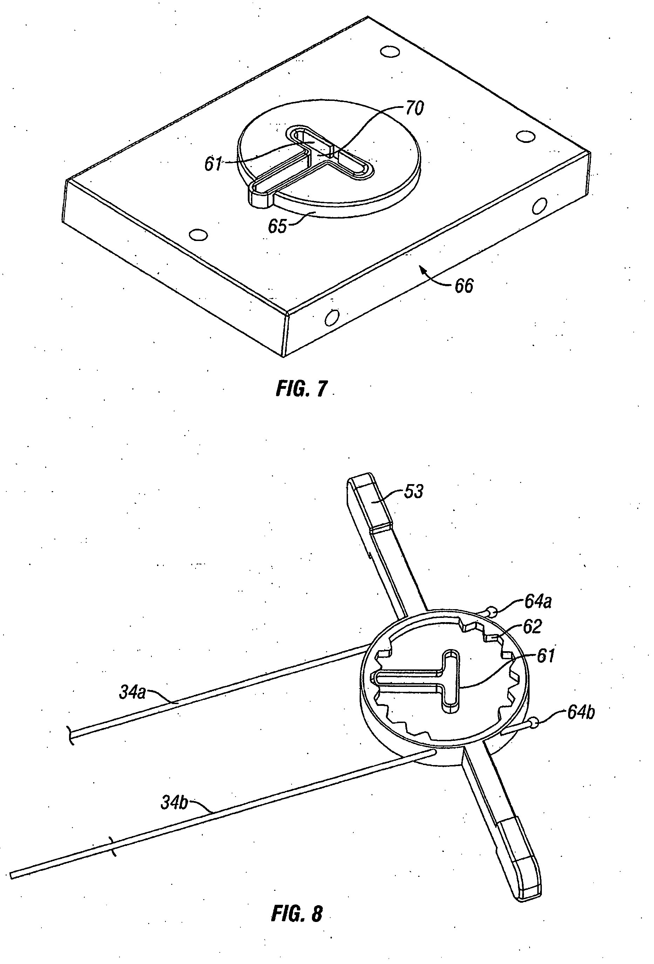

FIG. 7 is a perspective schematic view of the base portion of the articulation control 66 showing the spring mechanism 61 sitting in a recess 70 of the ring-like projection 65.

FIG. 8 is a schematic perspective view of the articulation control 53 showing the detents 62 in greater detail.

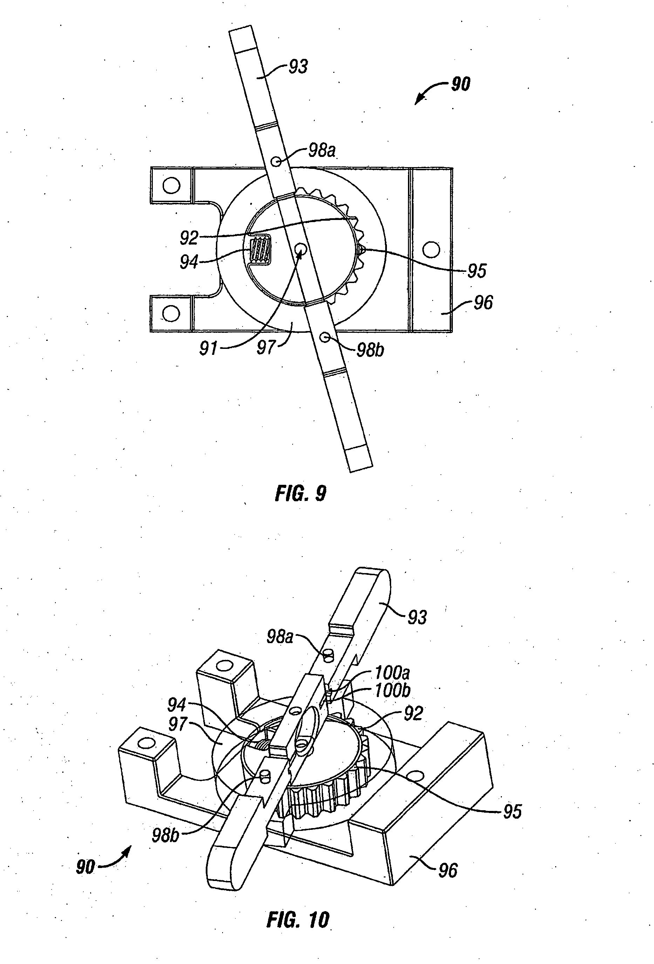

FIG. 9 shows an alternative embodiment, in which an articulation control 93 includes a plurality of detents 92 formed in a detent indexing disc 97. Operation of the control 93 causes rotation about a pivot point 91 and engages a step ball 95 into one of a plurality of detents 92 formed in the indexing ring. A ball plunger mechanism 94 maintains bias on the step ball 95. The indexing control 93 includes a pair of attachment points 98a/98b, discussed in greater detail below.

FIG. 10 is a perspective view of the index control mechanism for the articulation mechanism in the laparoscopic device. As shown in FIG. 10, a pair of grooves 100a/100b are provided for receiving control cables (not shown).

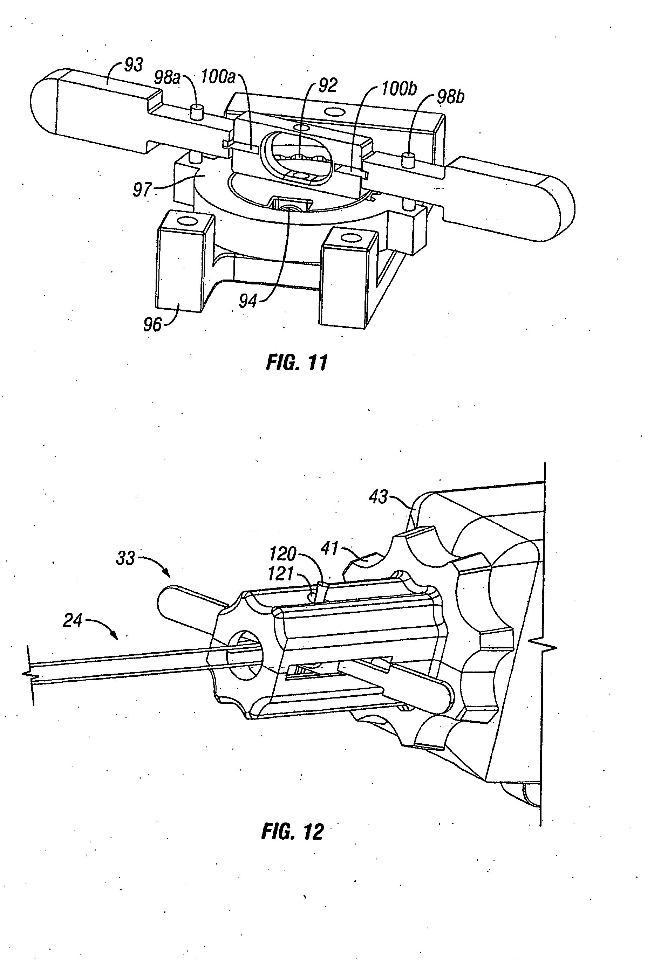

FIG. 11 is a further perspective view of the control mechanism for the articulation wrist in a laparoscopic device. FIG. 11 shows clearly the arrangement of the articulation control 93 in connection with the indexing ring 97, and in particular shows the attachment there between a pair of pins 98a/98b.

FIG. 12 is a further embodiment showing an indexing mechanism comprised of an indexing pin 120, which is engaged in a slot 121.

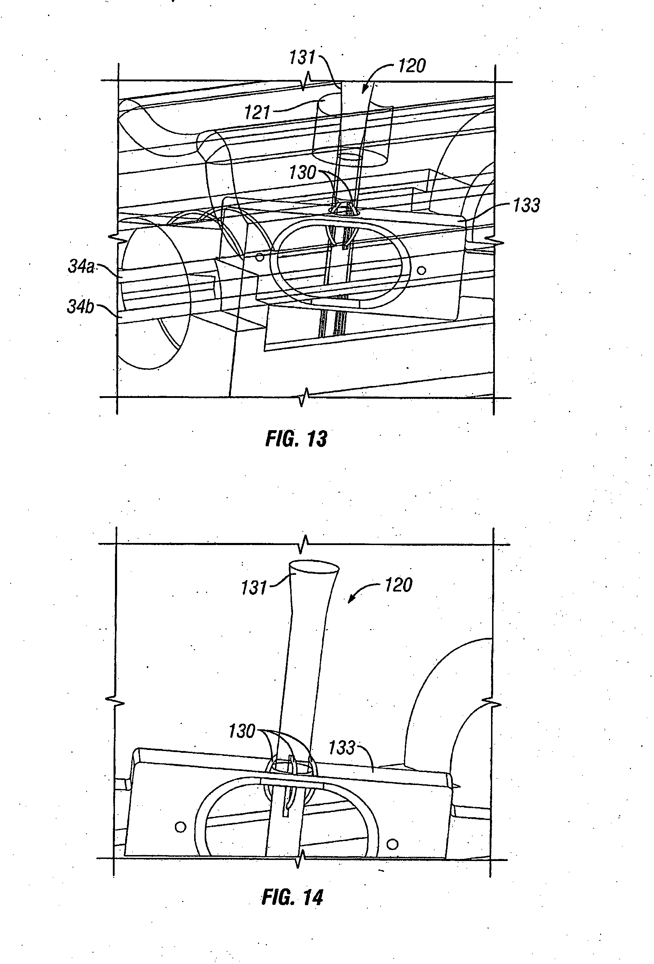

FIG. 13 is a cutaway perspective view showing the indexing pin 120 comprising a head portion 131 and a plurality of flared portions 130 which engage or disengage with a locking block 133. Accordingly, this embodiment comprises a jam lock in which depression of the pin 120 jams the flared portion of the pin 130 into the block 133 and thus prevents rotation of the actuation control mechanism.

FIG. 14 is a detailed view of the jam mechanism showing the pin 120, head 131, and flares 130 in greater detail.

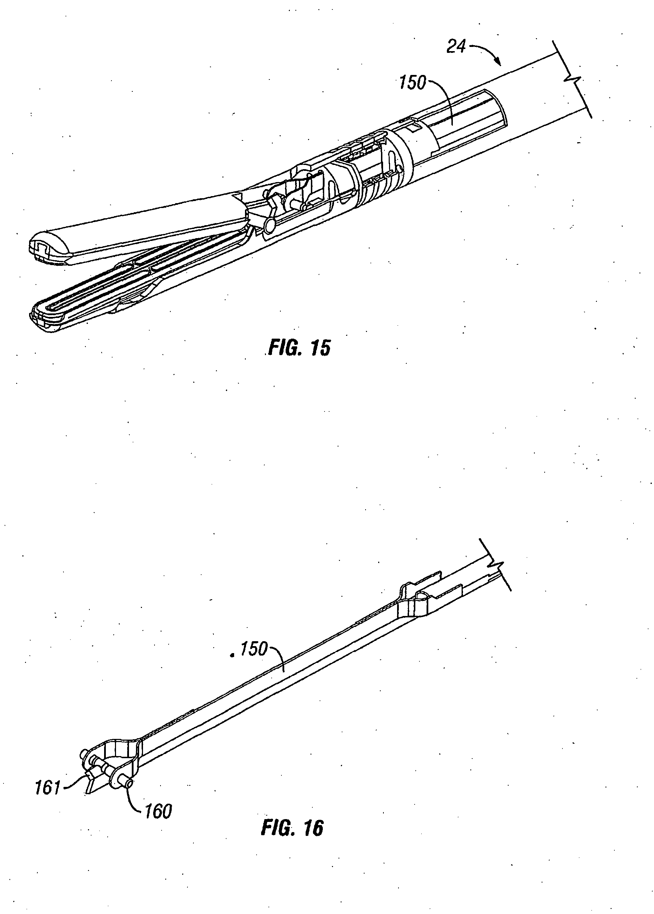

FIG. 15 is a perspective, partially cutaway view of a laparoscopic device, showing a drive member. The drive members may be made of a round wire (stainless steel or Nitinol), using tightly wound coil springs for support. The drive members may also be flat stainless steel bands 150, as shown in FIGS. 15 and 16. FIG. 15 shows the wrist section of the device, while FIG. 16 shows only the components of interest; i.e:, the jaw activating band 150, the closing pin 160, and cutting blade 161. This embodiment replaced the round wire with flat bands and supports the bands using the internal structure of the vertebrae. Other embodiments may use flat polymer bands to provide additional support. These bands could be either PTFE (Teflon(R)) or FEP. The support structure may also involve PTFE or FEP shrink tubing over the blade and/or the jaw actuation band.

## Claims

1. A laparoscopic grasping instrument, comprising:
an elongated shaft (24) having a set of opposing jaws (25) associated with a distal end thereof and a handle (43, 44) associated with a proximal end thereof;
an articulation wrist (18, 19) positioned between said shaft and said jaw set for effecting movement of said jaws relative to said shaft as desired, both left and right, from centerline,:
an articulation actuator (53) associated with said handle (43, 44);
at least one force transfer member (34a, 34b) for translating user operation of said actuator into movement of said jaws to control the degree of articulation of said instrument; and
a further articulation wrist (22) comprising a set of pivotal vertebra that are connected to each other, wherein each vertebra is adapted to pivot left and right from centerline in relation to any of said shaft and said jaw set,
**characterized by**
a locking mechanism for preventing motion of said further articulation wrist while a user performs other operations on said instrument,
a spring (61) having a shape that deflects when a force is applied thereto;
wherein said spring is housed within a circular carrier (65); and
wherein only the deflectable portion of said spring is accessible and protruding from said circular carrier;
a rotating member (65) having a circular portion removed from a pivot area; wherein said rotating member is adapted to fit over said circular carrier; and
a tooth pattern (62) that is removed from along an inner diameter of said circular portion of said rotating member, and comprises teeth;
said rotating member comprising arms (53) extending from a center body thereof to which said one or more force transfer members are attached;
wherein said spring protrudes into indentations created by said tooth pattern; and
wherein an angle of articulation of said further articulation wrist is controlled by predetermining distances between said teeth and a distance from an attachment point of said one or more force transfer member to a pivot point.

2. The instrument of claim 1, further comprising:
a jaw actuation member.

3. The instrument of claim 1 or 2, further comprising:
a cutter; and
a cutter driving member.

4. The instrument of claim 1, 2 or 3, further comprising:
a bendable support for said force transfer member comprising a tightly wound coil spring housed within said wrist joints to route said drive member.

5. The instrument of anyone of claims 1 to 4, said locking mechanism further comprising:
an indexing mechanism with which said user can index and choose a necessary amount of jaw angle between preset angles.

6. The instrument of anyone of claims 1 to 5, said force transfer member comprising:
one or more wires or cables that run down both sides of the further articulation wrist for transferring forces from said actuator to said wrist.

7. The instrument of anyone of the preceding claims, said actuator comprising:
a rotating assembly that receives said one or more force transfer members from said wrist;
wherein said rotating assembly is mounted to pivot about a pivot point at said handle; and
wherein said actuator is configured for concentric rotation about said pivot point;
wherein length-wise motion of said one or more force transfer members along said shaft is controlled, based upon a distance from said pivot point to an attachment point of said one or more force transfer members.

8. The instrument of claim 7, wherein an angle of articulation is controlled by a distance that said one or more force transfer members move, which is predetermined by said wrist geometry.

9. The instrument of anyone of claims 1 to 8, said locking mechanism further comprising:
a wing is mounted on top of a rotating member;
wherein said wing is adapted to be manipulated to control rotation of said rotating member around a circular carrier.

10. The instrument of anyone of claims 1 to 9, said jaw set further comprising:
at least one set of electrodes for receiving an electric charge and for imparting said charge to an organ or tissue during an electrocautery procedure.

11. The instrument of any one of claims 1 to 10, further comprising an instrument drive member that effects selected instrument operation in response to selected user instrument activation.

12. The instrument of claim 11, said drive member comprising a flat band.

13. The instrument of claim 11, said instrument operation comprising movement of a blade.

## Patentansprüche

1. Laparoskopisches Greifinstrument, mit:
einem langgestreckten Schaft (24), der einen Satz von gegenüberliegenden Backen (25), die mit einem distalen Ende desselben verknüpft sind, und einen Handgriff (43, 44) aufweist, der einem proximalen Ende desselben zugeordnet ist;
einem Artikulationsgelenk (18, 19) bzw. Handgelenk, das zwischen dem Schaft und dem Satz von Backen für die Bewirkung einer Bewegung der Backen relativ zu dem Schaft je nach Wunsch sowohl nach links als auch nach rechts von einer Mittellinie positioniert ist,
einem Gelenkaktuator (53), der mit dem Handgriff (43, 44) verknüpft ist;
mindestens einem Kraftübertragungselement (34a, 34b) für die Umsetzung einer Benutzerbetätigung des Aktuators in eine Bewegung der Backen, um das Ausmaß der Verschwenkung des Instruments zu steuern; und
einem weiteren Artikulationsgelenk (22), das einen Satz von verschwenkbaren Wirbeln aufweist, die miteinander verbunden sind, wobei jeder Wirbel dazu ausgelegt ist, nach links und rechts von einer Mittellinie in Relation zu jedem von dem Schaft und dem Backensatz zu schwenken,
**gekennzeichnet durch**
einen Verriegelungsmechanismus für die Verhinderung einer Bewegung des weiteren Artikulationsgelenks, während ein Benutzer andere Betätigungen an dem Instrument ausführt,
eine Feder (61), die eine Gestalt aufweist, die sich verbiegt, wenn eine Kraft auf sie ausgeübt wird;
wobei die besagte Feder innerhalb eines kreisförmigen Trägers (65) untergebracht ist, und
wobei lediglich der verbiegbare Abschnitt der besagten Feder zugänglich ist und von dem kreisförmigen Träger vorsteht;
ein drehendes Element (65), das einen kreisförmigen Abschnitt aufweist, der von einem Schwenkbereich entfernt ist, wobei das drehende Element dazu angepasst ist, über den kreisförmigen Träger zu passen; und
ein Zahnmuster (62), das von entlang eines Innendurchmessers des kreisförmigen Abschnitts des drehenden Elements entfernt ist sowie Zähne aufweist;
wobei das drehende Element Arme (53) aufweist, die sich von einem Mittelkörper desselben erstrecken, an denen das eine oder die mehreren Kraftübertragungselemente angebracht sind;
wobei die Feder in Vertiefungen hinein ragt, die **durch** das Zahnmuster geschaffen sind; und
wobei ein Winkel der Artikulation des weiteren Artikulationsgelenks **durch** vorbestimmte Abstände zwischen den Zähnen und einem Abstand zu einem Anbringungspunkt des einen oder der mehreren Kraftübertragungselemente an einem Schwenkpunkt gesteuert ist.

2. Instrument nach Anspruch 1, das weiterhin umfasst:
ein Backenbetätigungselement.

3. Instrument nach Anspruch 1 oder 2, das weiterhin aufweist:
ein Schneidelement; und
ein Schneidelement-Antriebselement.

4. Instrument nach Anspruch 1, 2 oder 3, das weiterhin umfasst:
eine biegbare Halterung für das Kraftübertragungselement, das eine eng gewickelte Schraubenfeder umfasst, die innerhalb der Gelenkverbindungen für die Führung des Antriebselements untergebracht ist.

5. Instrument nach einem beliebigen der Ansprüche 1 bis 4, wobei der Verriegelungsmechanismus weiterhin umfasst:
einen Indexiermechanismus, durch den der Benutzer ein notwendiges Ausmaß des Backenwinkels zwischen voreingestellten Winkeln indexieren und wählen kann.

6. Instrument nach einem beliebigen der Ansprüche 1 bis 5, wobei das Kraftübertragungselement umfasst:
einen oder mehrere Drähte oder Kabel, die an beiden Seiten des weiteren Artikulationsgelenks für die Übertragung von Kräften von dem Aktuator zu dem Gelenk entlang verlaufen.

7. Instrument nach einem beliebigen der vorhergehenden Ansprüche, wobei der Aktuator umfasst:
eine drehende Anordnung, die das eine oder die mehreren Kraftübertragungselemente von dem Gelenk aufnimmt;
wobei die drehende Anordnung so montiert ist, dass sie um einen Schwenkpunkt an dem Handgriff schwenkt; und
wobei der Aktuator für eine konzentrische Drehung um den besagten Schwenkpunkt konfiguriert ist;
wobei eine in Längsrichtung erfolgende Bewegung des einen oder der mehreren Kraftübertragungselemente entlang des Schafts auf der Basis eines Abstands von dem besagten Schwenkpunkt zu einem Befestigungspunkt des einen oder der mehreren Kraftübertragungselemente gesteuert ist.

8. Instrument nach Anspruch 7, bei dem ein Winkel der Artikulation durch einen Abstand gesteuert wird, um den sich das eine oder die mehreren Kraftübertragungselemente bewegen, der durch die Gelenkgeometrie vorbestimmt ist.

9. Instrument nach einem beliebigen der Ansprüche 1 bis 8, wobei der Verriegelungsmechanismus weiterhin umfasst:
einen Flügel, der an der Oberseite eines drehenden Elements montiert ist,
wobei der Flügel dazu ausgelegt ist, manipuliert zu werden, um eine Drehung des drehenden Elements um einen kreisförmigen Träger zu steuern.

10. Instrument nach einem beliebigen der Ansprüche 1 bis 9, wobei der Gelenksatz weiterhin umfasst:
mindestens einen Satz von Elektroden für die Aufnahme einer elektrischen Ladung und für die Aufbringung der Ladung auf ein Organ oder ein Gewebe während einer Elektrokauterisierungsprozedur.

11. Instrument nach einem beliebigen der Ansprüche 1 bis 10, das weiterhin ein Instrumentantriebselement umfasst, das eine ausgewählte Instrumentenbetätigung als Reaktion auf eine ausgewählte benutzerseitige Aktivierung des Instruments bewirkt.

12. Instrument nach Anspruch 11, wobei das Antriebselement ein flaches Band ist.

13. Instrument nach Anspruch 11, wobei die Instrumentenbetätigung eine Bewegung einer Klinge umfasst.

## Revendications

1. Instrument de saisie laparoscopique, comprenant :
un arbre allongé (24) ayant un ensemble de mâchoires opposées (25) associé à son extrémité distale et une poignée (43, 44) associée à son extrémité proximale ;
un poignet d'articulation (18, 19) positionné entre ledit arbre et ledit ensemble de mâchoires pour effectuer le mouvement desdites mâchoires par rapport audit arbre, si nécessaire, à la fois à gauche et à droite, depuis l'axe central,
un actionneur d'articulation (53) associé à ladite poignée (43, 44) ;
au moins un élément de transfert de force (34a, 34b) pour translater l'opération dudit actionneur réalisée par l'utilisateur, en mouvement desdites mâchoires afin de réguler le degré d'articulation dudit instrument ; et
un autre poignet d'articulation (22) comprenant un ensemble de vertèbres pivotantes qui sont raccordées entre elles, dans lequel chaque vertèbre est adaptée pour pivoter à gauche et à droite depuis l'axe central par rapport à l'un quelconque parmi ledit arbre et ledit ensemble de mâchoires,
**caractérisé par**
un mécanisme de verrouillage pour empêcher le mouvement dudit poignet d'articulation supplémentaire alors qu'un utilisateur réalise d'autres opérations sur ledit instrument,
un ressort (61) ayant une forme qui dévie lorsqu'une force est appliquée sur ce dernier ;
dans lequel ledit ressort est logé à l'intérieur du support circulaire (65) ; et
dans lequel seule la partie pouvant être déviée dudit ressort est accessible et faisant saillie dudit support circulaire ;
un élément rotatif (65) ayant une partie circulaire retirée d'une zone de pivot ;
dans lequel ledit élément rotatif est adapté pour se monter sur ledit support circulaire ; et
un motif de dent (62) qui est retiré le long d'un diamètre interne de ladite partie circulaire dudit élément rotatif, et comprend des dents ;
ledit élément rotatif comprenant des bras (53) s'étendant à partir de son corps central auquel lesdits un ou plusieurs éléments de transfert de force sont fixés ;
dans lequel ledit ressort fait saillie dans des dentelures créées par ledit motif de dent ; et
dans lequel un angle d'articulation dudit poignet d'articulation supplémentaire est contrôlé par des distances de prédétermination entre lesdites dents et une distance à partir d'un point de fixation desdits un ou plusieurs éléments de transfert de force jusqu'à un point de pivot.

2. Instrument selon la revendication 1, comprenant en outre :
un élément d'actionnement de mâchoire.

3. Instrument selon la revendication 1 ou 2, comprenant en outre :
un dispositif de coupe ; et
un élément d'entraînement de dispositif de coupe.

4. Instrument selon la revendication 1, 2 ou 3, comprenant en outre :
un support pliable pour ledit élément de transfert de force comprenant un ressort hélicoïdal enroulé de manière serrée, logé à l'intérieur desdites articulations de poignet pour diriger ledit élément d'entraînement.

5. Instrument selon l'une quelconque des revendications 1 à 4, ledit mécanisme de verrouillage comprenant en outre :
un mécanisme d'indexation avec lequel ledit utilisateur peut indexer et choisir une quantité nécessaire d'angle de mâchoire entre des angles prédéterminés.

6. Instrument selon l'une quelconque des revendications 1 à 5, ledit élément de transfert de force comprenant :
un ou plusieurs fils ou câbles qui descendent des deux côtés du poignet d'articulation supplémentaire pour transférer des forces dudit actionneur audit poignet.

7. Instrument selon l'une quelconque des revendications précédentes, ledit actionneur comprenant :
un ensemble de rotation qui reçoit lesdits un ou plusieurs éléments de transfert de force dudit poignet ;
dans lequel ledit ensemble de rotation est monté pour pivoter autour d'un point de pivot au niveau de ladite poignée; et
dans lequel ledit actionneur est configuré pour tourner de manière concentrique autour dudit point de pivot ;
dans lequel le mouvement dans le sens de la longueur desdits un ou plusieurs éléments de transfert de force le long dudit arbre est contrôlé, en fonction d'une distance allant dudit point de pivot à un point de fixation desdits un ou plusieurs éléments de transfert de force.

8. Instrument selon la revendication 7, dans lequel un angle d'articulation est contrôlé par une distance sur laquelle lesdits un ou plusieurs éléments de transfert de force se déplacent, qui est prédéterminée par ladite géométrie du poignet.

9. Instrument selon l'une quelconque des revendications 1 à 8, ledit mécanisme de verrouillage comprenant en outre :
une aile qui est montée sur le dessus d'un élément rotatif ;
dans lequel ladite aile est adaptée pour être manipulée afin de contrôler la rotation dudit élément rotatif autour d'un support circulaire.

10. Instrument selon l'une quelconque des revendications 1 à 9, ledit ensemble de mâchoires comprenant en outre :
au moins un ensemble d'électrodes pour recevoir une charge électrique et pour communiquer ladite charge à un organe ou tissu pendant une procédure d'électrocautère.

11. Instrument selon l'une quelconque des revendications 1 à 10, comprenant en outre un élément d'entraînement d'instrument qui effectue l'opération d'instrument sélectionnée en réponse à l'activation d'instrument d'utilisateur sélectionnée.

12. Instrument selon la revendication 11, ledit élément d'entraînement comprenant une bande plate.

13. Instrument selon la revendication 11, ladite opération d'instrument comprenant le mouvement d'une lame.
